# EUROPÄISCHE PATENTSCHRIFT

(11) **EP 2 818 138 B1**
(45) Veröffentlichungstag und Bekanntmachung des Hinweises auf die Patenterteilung: **08.03.2017**
(21) Anmeldenummer: 14173374.1
(22) Anmeldetag: 23.06.2014
(51) Int. Cl.: A61F 2/30

(54) **Verfahren zur Herstellung von Spacern und Vorrichtung hierzu**
Method for the production of spacers and device for same
Procédé de fabrication d'espaceurs et dispositif correspondant

(30) Priorität: 26.06.2013 DE 102013010593
(43) Veröffentlichungstag der Anmeldung: 31.12.2014
(73) Patentinhaber: Heraeus Medical GmbH, 61273 Wehrheim (DE)
(72) Erfinder: Vogt, Sebastian, 99092 Erfurt (DE)
(74) Vertreter: Schultheiss & Sterzel Patentanwälte PartG mbB

(56) Entgegenhaltungen:
- EP-A1- 2 526 900
- EP-A1- 2 532 323
- WO-A1-2008/088869
- US-A1- 2009 157 189

## Beschreibung

Die Erfindung betrifft ein Verfahren zur Herstellung von Spacern aus einem Knochenzement und eine Vorrichtung zur Herstellung von Spacern aus einem Knochenzement mit einem solchen Verfahren.

Gegenstand der Erfindung ist somit ein Verfahren zur Herstellung von weitgehend porenfreien Polymethylmethacrylat-Spacern (PMMA-Spacern), die als temporärer Platzhalter im Rahmen von zweizeitigen septischen Revisionen von Gelenkendoprothesen verwendet werden.

Gelenkendoprothesen haben gegenwärtig eine Standzeit von mehreren Jahren, zum Beispiel bei zementierten Hüftgelenkprothesen im Durchschnitt grösser als zehn bis fünfzehn Jahre. Es gibt jedoch unerwünschte Lockerungen der Gelenkendoprothesen, die vor dem Erreichen der üblichen Standzeiten auftreten. Es wird dabei die septische und die aseptische Lockerung unterschieden. Bei der aseptischen Lockerung lassen sich bisher keine mikrobiellen Keime nachweisen. Die Ursachen der aseptischen Lockerungen können vielfältig sein. Häufig sind aseptische Lockerungen auf Abrieb an den Gleitflächen der Gelenkendoprothesen zurückzuführen. Bei der septischen Lockerung wird der Lockerungsprozess durch mikrobielle Keime hervorgerufen. Man unterscheidet dabei in Abhängigkeit vom zeitlichen Auftreten frühe und späte Infektionen. Die septische Lockerung ist eine sehr ernste Erkrankung für den Patienten, die zusätzlich mit sehr hohen Kosten verbunden ist. Sowohl bei der aseptischen als auch der septischen Lockerung wird üblicherweise eine Revision vorgenommen. Man unterscheidet einzeitige und die zweizeitige Revisionen. Bei septischen Lockerungen werden sehr häufig zweizeitige Revisionen vorgenommen.

Bei der zweizeitigen Revision wird in einer ersten Operation (OP) die infizierte Gelenkendoprothese entfernt, es erfolgt ein Debridement und anschließend wird ein temporärer Platzhalter, ein so genannter Spacer, eingesetzt. Dieser Spacer füllt für einige Wochen den Raum der zuvor revidierten Endoprothese aus, bis die vorliegende Infektion abgeklungen ist. Diese Platzhalterfunktion ist sehr wichtig, um eine Atrophie der Muskulatur in dieser Zeit wirksam zu verhindern und um eine Stabilisierung der Resektionssituation zu erreichen. Man unterscheidet dann nichtartikulierende und artikulierende Spacer. Artikulierende Spacer oder Gelenkspacer bilden die Gelenkfunktion nach und erlauben eine gewisse Beweglichkeit der betroffenen Gliedmaßen. Es ist dadurch möglich, die Patienten frühzeitig zu mobilisieren. Artikulierende Gelenkspacer stellen heute den Stand der Technik dar. Der Spacer wird in einer zweiten OP entfernt, es wird nochmals debridiert und dann wird eine zementierte oder auch zementfreie Revisions-Gelenkendoprothese implantiert.

Die Verwendung von Spacern geht ursprünglich auf Hovelius und Josefsson zurück (Hovelius L, Josefsson G (1979), "An alternative method for exchange operation of infected arthroplasty", Acta Orthop. Scand. 50: 93-96). Weitere frühe Arbeiten zu Spacern stammen von Younger (Younger AS, Duncan CP, Masri BA, McGraw RW (1997), "The outcome of two-stage arthroplasty using a custom-made interval spacer to treat the infected hip", J. Arthroplasty 12: 615-623), Jones (Jones WA, Wroblewski BM (1989), "Salvage of failed total knee arthroplasty: the 'beefburger' procedure", J. Bone Joint Surg. Br. 71: 856-857.) und Cohen (Cohen JC, Hozack WJ, Cuckler JM, Booth RE Jr (1988), "Two-stage reimplantation of septic total knee arthroplasty, Report of three cases using an antibiotic-PMMA spacer block", J. Arthroplasty 3: 369-377). Von McPherson stammt die Konzeption, dass Spacer ausschließlich aus Knochenzement hergestellt werden können (McPherson EJ, Lewonowski K, Dorr LD (1995), "Techniques in arthroplasty. Use of an articulated PMMA spacer in the infected total knee arthroplasty", J. Arthroplasty 10: 87-89).

Es gibt auf dem Markt mit Antibiotika ausgerüstete Spacer zum temporären Ersatz von Knie-, Hüft- und Schultergelenkendoprothesen. Nachteilig daran ist jedoch, dass die enthaltenen Antibiotika vorgegeben sind und nicht entsprechend dem Antibiogramm der vorgefundenen mikrobiellen Keime speziell angepasst werden können.

Spacer werden häufig während der Operation vom Arzt selbst per Hand aus Zementteig geformt oder unter Verwendung von elastischen Silikonformen selbst gegossen. Alternativ dazu gibt es seit mehreren Jahren auch industriell hergestellt Spacer, die in verschiedenen StandardGrößen angeboten werden. Diese vorgefertigten Spacer können vom Arzt ohne größere Vorbereitungsarbeiten direkt implantiert werden, wobei die Spacer üblicherweise mit Polymethylmethacrylat-Knochenzement am Knochengewebe fixiert wird. Der Vorteil der industriell hergestellten Spacer besteht darin, dass die Oberflächenqualität der Gleitflächen der vorgefertigten Spacer im Allgemeinen deutlich besser ist als die der intra-operativ gefertigten Spacer. Die Gleitflächen sind die Oberflächen artikulierender Spacerteile, die aufeinander gleiten, beziehungsweise aufeinander abrollen, um die Funktion eines Gelenks durch den Spacer nachzubilden. Weiterhin kann durch die Verwendung von vorgefertigten Spacern wertvolle OP-Zeit gespart werden und der gesamte OP-Ablauf ist deutlich vereinfacht, weil der zeitaufwendige und arbeitsaufwendige Schritt der Herstellung der Spacer entfällt.

Die US 2009/0157189 A1 offenbart ein Verfahren zur Herstellung eines Spacers mit einer Gießform, bei dem ein Zementteig in die Gießform gefüllt wird, der Zementteig in der Gießform aushärtet und nach der Aushärtung des Zementteigs die Gießform von dem ausgehärteten Spacer getrennt wird.

Das Gießen von Spacern mit Polymethylmethacrylat-Knochenzement unter Verwendung von elastischen Gießformen, wie zum Beispiel von Silikonformen, führt zu Spacern mit relativ guter Oberflächenqualität. Problematisch ist jedoch für eine industrielle Herstellung, dass Gießformen aus gummielastischem Material relativ schnell verschleißen und daher nicht für die Herstellung größerer Stückzahlen von Spacern geeignet sind. Silikonformen sind weiterhin in der Fertigung kostenintensiv. Daher ist es sinnvoll, weitgehend verschleißfeste Gießformen einzusetzen.

Eigene Gießversuche zeigten bei der Verwendung von verschleißfesten Gießformen aus Metall oder nicht gummielastischen Kunststoffen und Polymethylmethacrylat-Knochenzementteig bei Raumtemperatur, dass es an der Oberfläche der Spacer zur Bildung von unregelmäßigen porenartigen Vertiefungen kommt. Diese Poren beziehungsweise Störungen der Oberfläche sind besonders störend im Bereich der Gleitflächen. Poren oder auch andere Unebenheiten der Gleitflächen können während des Zeitraums der Implantation im Patienten zu einer verstärkten Bildung von Abrieb führen, der gegebenenfalls später Entzündungsprozesse begünstigen oder auch auslösen kann, wenn der Abrieb nicht vollständig bei der Explantation der Spacer entfernt wird. Weiterhin können Poren und andere Störungen der Oberfläche als Rissansatzstellen wirken und dadurch die mechanische Stabilität der Spacer beeinträchtigen. Es ist möglich die Poren oder auch andere Störungen der Oberfläche durch Schleifen und Polieren zu vermindern oder zu beseitigen. Jedoch ist diese Nacharbeit zeit- und kostenintensiv.

Die Aufgabe der Erfindung besteht also darin, die Nachteile des Stands der Technik zu überwinden. Insbesondere sollen ein Verfahren und eine Vorrichtung zur Herstellung von Spacern aus Knochenzement, insbesondere auch Polymethylmethacrylat-Knochenzement entwickelt werden, mit dem unter Verwendung von verschleißbeständigen, formbeständigen Gießformen Spacer im industriellen Maßstab so hergestellt werden können, wobei die Bildung von Poren oder anderen Oberflächenstörungen weitgehend ausgeschlossen ist.

Die Aufgaben der Erfindung werden gelöst durch ein Verfahren zur Herstellung von Spacern aus einem Knochenzement aufweisend die folgenden chronologischen Schritte:
A) eine Gießform wird mittels einer Temperiereinrichtung auf eine erste Temperatur temperiert,
B) ein Zementteig mit einer Temperatur kleiner als die erste Temperatur der temperierten Gießform wird in die temperierte Gießform eingefüllt,
C) der Zementteig härtet innerhalb der Gießform zu einem Spacer aus und
D) nach der Aushärtung des Spacers wird die Gießform vom Spacer getrennt.

Die Gießform kann während des Aushärtens des Knochenzements weiter temperiert werden. Wenn die Wärmekapazität der Gießform ausreichend groß ist, kann es aber auch ausreichend sein, wenn die Gießform beim Einfüllen des Knochenzements temperiert war. Für letzteres reicht es beispielsweise aus, wenn eine Gießform aus Edelstahl eine Wanddicke von mindestens einem Millimeter aufweist.

Bei einem erfindungsgemäßen Verfahren kann vorgesehen sein, dass als Zementteig ein Zementteig aus Polymethylmethacrylat (PMMA) verwendet wird, bevorzugt ein durch Vakuumeinwirkung entgaster Zementteig enthaltend Polymethylmethacrylat verwendet wird, und der erzeugte Spacer aus Polymethylmethacrylat besteht.

Diese Knochenzemente sind zur Umsetzung des erfindungsgemäßen Verfahrens besonders gut geeignet. Zudem sind die aus diesen Knochenzementen gebildeten Spacer besonders gut biokompatibel.

Ferner kann vorgesehen sein, dass zumindest die den Spacer formende Oberfläche der Gießform zu wenigstens 90%, bevorzugt zu wenigstens 99% aus einem nicht elastischen Material besteht, bevorzugt aus Polyethylen, Polypropylen, Polyamid und/oder Edelstahl, besonders bevorzugt aus dem Edelstahl 1.4401 und/oder dem Edelstahl 1.4404.

Diese Materialien sind ausreichend Formstabil und lassen sich gut auch bei einer Mehrfachverwendung von den erzeugten Spacern beziehungsweise Spacerteilen lösen.

Mit einer bevorzugten Weiterbildung der Erfindung wird vorgeschlagen, dass der Zementteig mit einer Temperatur, die zumindest 10 °C kleiner als die erste Temperatur des temperierten Gießform ist, in die temperierte Gießform eingefüllt wird, bevorzugt mit einer Temperatur, die zumindest 20 °C kleiner, besonders bevorzugt 20 °C bis 50 °C kleiner ist als die erste Temperatur des temperierten Gießform.

Diese Temperaturdifferenzen stellen sicher, dass eine gerichtete Aushärtung des Knochenzements von der Inneren Oberfläche der Gießform aus erfolgt.

Es kann auch vorgesehen sein, dass die Gießform auf eine erste Temperatur zwischen 40 °C und 65 °C temperiert wird, bevorzugt zwischen 45 °C und 60 °C, besonders bevorzugt zwischen 50 °C und 55 °C.

Diese Temperaturen sind zum Starten des Polymerisationsvorgangs der bevorzugt verwendeten PMMA-Knochenzemente besonders gut geeignet. Daher sind diese Temperaturen für die Gießformen besonders bevorzugt.

Des Weiteren ist es besonders vorteilhaft, wenn vorgesehen ist, dass der Zementteig mit einer Temperatur zwischen -20 °C und 30 °C in die Gießform eingefüllt wird, bevorzugt zwischen -20 °C und 10 °C, besonders bevorzugt zwischen -20 °C und 0 °C.

Hierdurch wird eine Aushärtung des Knochenzements, insbesondere eine Polymerisation des PMMA-Knochenzements im Inneren reduziert, so dass die gerichtete Aushärtung von außen erfolgen wird.

Mit einer Weiterbildung der Erfindung wird vorgeschlagen, dass der Zementteig auf eine Temperatur kleiner als die erste Temperatur der temperierten Gießform gebracht wird, bevor er in die Gießform gefüllt wird.

Durch eine aktive Temperierung des Zementteigs ist der Ablauf des erfindungsgemäßen Verfahrens besser reproduzierbar, das Verfahren standardisierbar und damit einer Massenproduktion einfacher zugänglich.

Es kann bevorzugt auch vorgesehen sein, dass der Zementteig mit einem Volumen, das zumindest 2 Vol% größer als das Formnest der Gießform ist, bevorzugt zumindest 5 Vol% größer ist, in die Gießform eingefüllt wird, wobei das zusätzliche Volumen zumindest vor dem Aushärten des Knochenzements in einem Anguss der Gießform enthalten ist.

Hierdurch kann eine Schrumpfung des Knochenzements beim Aushärten ausgeglichen werden.

Des Weiteren kann erfindungsgemäß bevorzugt vorgesehen sein, dass in die Gießform zur Armierung des Spacers mindestens ein Stahlkern eingelegt wird, dessen Temperatur mindestens 10 °C niedriger ist als die erste Temperatur der temperierten Gießform, bevorzugt mindestens 30 °C niedriger als die erste Temperatur der temperierten Gießform ist.

Hierdurch wird eine Stabilisierung des Spacers beziehungsweise des Spacerteils erreicht. Durch die Temperierung des Stahlkerns wird verhindert, dass sich die Polymerisation beziehungsweise das Aushärten des Knochenzements vom Stahlkern aus nach Außen fortpflanzt. Bevorzugt hat der Stahlkern die gleiche oder eine niedrigere Temperatur als der Zementteig.

Dabei kann erfindungsgemäß vorgesehen sein, dass der mindestens eine Stahlkern durch Abstandhalter in der Gießform derart gelagert wird, dass der Abstand zwischen der Innenseite der Gießform und der Oberfläche des Stahlkern festgelegt ist, wobei die Abstandhalter eine Temperatur haben, die mindestens 10 °C niedriger ist als die erste Temperatur der temperierten Gießform, bevorzugt mindestens 30 °C niedriger als die erste Temperatur der temperierten Gießform ist.

Durch die Abstandhalter kann der Stahlkern oder können die Stahlkerne in einer definierten Position im Spacer angeordnet werden. Dabei wird durch die Temperierung der Abstandhalter erreicht, dass diese nicht als Keime beziehungsweise Startpunkte für die Polymerisation beziehungsweise das Aushärten des Knochenzements wirken. Bevorzugt haben die Abstandhalter die gleiche oder eine niedrigere Temperatur als der Zementteig.

Mit einer Weiterentwicklung des erfindungsgemäßen Verfahrens wird vorgeschlagen, dass der Knochenzement mindestens ein Antibiotikum und/oder ein Antiseptikum enthält, wobei bevorzugt zwei Antibiotika und/oder Antiseptika enthalten sind und ganz besonders bevorzugt drei Antibiotika und/oder Antiseptika enthalten sind.

Hierdurch ist der Spacer zum Bekämpfen von Infektionsherden geeignet.

Die der Erfindung zugrundeliegenden Aufgaben werden auch gelöst durch eine Vorrichtung zur Herstellung von Spacern aus einem Knochenzement mit einem solchen Verfahren aufweisend eine Gießform und eine Temperiereinrichtung zum Temperieren der Gießform, wobei die innere Oberfläche der Gießform zu wenigstens 80% ein Negativ der zu erzeugenden Oberfläche des Spacers aufweist.

Dabei kann vorgesehen sein, dass die Gießform aus zumindest zwei gegeneinander beweglichen Teilen besteht.

Hierdurch kann die Gießform leichter von dem fertig ausgehärteten Knochenzement gelöst werden.

Auch kann vorgesehen sein, dass die Vorrichtung ein Knochenzementreservoir und einen Einfüllstutzen aufweist, der mit dem Knochenzementreservoir verbunden ist und über den die Gießform mit Knochenzement befüllbar ist.

Hierdurch wird die Befüllung der Gießform mit dem Knochenzementteig erleichtert, insbesondere bei einer Automatisierung des gesamten Prozesses.

Des Weiteren kann vorgesehen sein, dass die Gießform zumindest einen Anguss aufweist, in den überschüssiger Knochenzement aufnehmbar ist, bevorzugt die Gießform zumindest einen Steiger, besonders bevorzugt mehrere Steiger zur Entlüftung des Inneren der Gießform an den am höchsten gelegenen Bereichen der Gießform aufweist.

Mit Hilfe des Anguss wird, ähnlich wie bei Gießformen für Metall, erreicht, dass auch bei einer Volumenverringerung des aushärtenden Knochenzements noch ein Reservoir des Knochenzements vorhanden ist, so dass sich keine Löcher in dem erzeugten Spacer bilden. Die Steiger dienen der Vermeidung von Lufteinschlüssen in der Gießform, die ansonsten zu Ausnehmungen im Spacer führen würden.

Schließlich kann vorgesehen sein, dass die Temperiereinrichtung eine elektrische Widerstandsheizung und/oder eine Flüssigkeitsheizung aufweist, die in den Wänden der Gießform enthalten und/oder die außen auf den Wänden der Gießform aufgebracht ist oder sind.

Alternativ kann die Temperiereinrichtung auch einfach von außen auf die Gießform aufliegen oder auf die Gießform aufstrahlen, um diese zu heizen und dadurch zu temperieren. Mit einer solchen Temperiereinrichtung kann eine Automatisierung des Verfahrens besonders einfach umgesetzt werden.

Der Erfindung liegt die überraschende Erkenntnis zugrunde, dass es durch die Temperierung der Gießform gelingt, eine gerichtete Aushärtung des Knochenzements zu erreichen, so dass die Oberfläche des erzeugten Spacers glatt und eben wird. Der Zementteig erstarrt bei dem erfindungsgemäßen Verfahren von außen nach innen, so dass bei einer Schrumpfung des aushärtenden Knochenzements fließfähiger Knochenzement die Schrumpfung ausgleichen kann und dadurch eine glatte Oberfläche des Spacers erzeugt wird.

Die Erfindung basiert dabei auf dem überraschenden Befund, dass beim Gießen mit Polymethylmethacrylat-Knochenzementteig Spacer mit porenfreien Oberflächen erhalten werden, wenn Gießformen aus nicht gummielastischem Material verwendet werden, die zuvor auf eine Temperatur von 40 °C oder größer temperiert wurden, wobei der in die Gießform einzubringende Polymethylmethacrylat-Knochenzementteig eine Temperatur von mindestens 10 °C niedriger besitzt als die Gießform.

Es wurde im Rahmen der vorliegenden Erfindung gefunden, dass die Bildung von Poren und anderen Oberflächenstörungen bei der Herstellung von Spacern in nicht gummielastischen, formstabilen Gießformen darauf zurück geführt werden kann, dass der Polymethylmethacrylat-Zementteig zuerst im Inneren des Spacers anfängt zu polymerisieren und dabei unter zunehmender Selbstbeschleunigung infolge der Freisetzung der Polymerisationsenthalpie polymerisiert. Dabei härtet der Polymethylmethacrylat-Zementteig von innen nach außen in Richtung der Gießformwand aus. Bei der Polymerisation setzt der Polymerisationsschrumpf ein. Das bedeutet, dass der aushärtende Zementteig von der Gießformwand zurückgezogen wird. Dabei reißt der Zementteig ungleichmäßig ab, wodurch Poren und andere Oberflächenstörungen ausgebildet werden. Der Anguss ist üblicherweise in der Mitte der Gießform angeordnet. Dadurch, dass die Polymerisation von innen nach außen erfolgt, kann kein Zementteig aus dem Anguss nachgezogen werden, um den Polymerisationsschrumpf zu kompensieren, weil der zentrale, mittige Bereich des Zementteigs um den Anguss als erstes polymerisiert und damit weiter ausgehärtet ist als der Zementteig im Bereich der Gießformwand.

Die radikalische Polymerisation des Knochenzementteigs ist stark temperaturabhängig. Das bedeutet, je höher die Temperatur ist, umso schneller härtet der Polymethylmethacrylat-Knochenzementteig aus. Die Idee der Erfindung besteht daher darin, die Gießform so zu erwärmen, dass die Gießform eine deutlich höhere Temperatur als der zu vergießende Polymethylmethacrylat-Knochenzementteig hat. Dadurch wird die Polymerisation an der Innenseite der Gießform beschleunigt. Somit polymerisiert der Zementteig von außen nach innen. Das bedeutet, der Polymerisationsschrumpf erfolgt von außen nach innen. Dadurch kann der schrumpfende Polymethylmethacrylat-Knochenzementteig aus dem Anguss und/oder den Steigern noch fließfähigen Polymethylmethacrylat-Knochenzementteig nachziehen. Es werden dadurch Spacer mit glatter porenfreier Oberfläche gebildet.

Die exotherme radikalische Polymerisation des Knochenzementteigs erzeugt selber die Wärme, die zu einem Fortschreiten der Polymerisation führt. Die Polymerisation zündet durch die Temperierung der Gießform an den Innenwänden der Gießform und pflanzt sich durch die Freisetzung von Wärme bei der Polymerisation ins Innere des polymerisierenden Spacers fort. Deswegen ist es auch nicht notwendig, die Gießform fortwährend zu temperieren, da die Wärmeenergie aus der Gießform bildlich gesprochen nur als Zündfunke für den Beginn der sich selbst erhaltenden Polymerisationsreaktion benötigt wird.

Die Erfindung kann beispielsweise realisiert werden durch ein Verfahren zur Herstellung von Polymethylmethacrylat-Spacern, bei dem in eine mindestens einteilige, auf 40 °C bis 65 °C temperierte Gießform, die mindestens ein Anguss enthält und die aus einem nicht gummielastischen Material besteht, ein durch Vakuumeinwirkung entgaster Polymethylmethacrylat-Zementteig gefüllt wird, dessen Temperatur mindestens 10 °C niedriger ist als die Temperatur der Gießform, wobei das Volumen des Polymethylmethacrylat-Zementteigs mindestens 2 Volumenprozent größer ist als das Volumen des Formnests, anschließend die Aushärtung innerhalb der Gießform erfolgt und die Gießform vom Polymethylmethacrylat-Spacer erst nach der Aushärtung des Polymethylmethacrylat-Spacers abgetrennt wird.

Im Folgenden werden weitere Ausführungsbeispiele der Erfindung anhand von vier schematisch dargestellten Figuren erläutert, ohne jedoch dabei die Erfindung zu beschränken. Dabei zeigt:
- Figur 1:: eine schematische perspektivische Ansicht eines unteren Teils einer erfindungsgemäßen Gießform zur Herstellung einer Tibia-Komponente eines Kniespacers;
- Figur 2:: eine schematische Querschnittansicht eines oberen Teils einer erfindungsgemäßen Gießform zur Herstellung einer Tibia-Komponente eines Kniespacers;
- Figur 3:: eine Abbildung einer Tibia-Komponente eines Kniespacers hergestellt mit einem nicht erfindungsgemäßen Verfahren; und
- Figur 4:: eine Abbildung einer Tibia-Komponente eines Kniespacers hergestellt mit einem erfindungsgemäßen Verfahren.

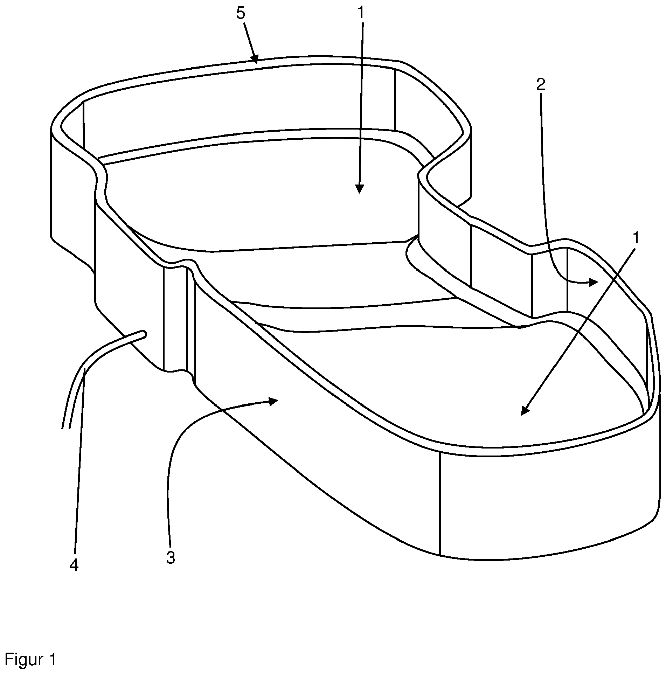

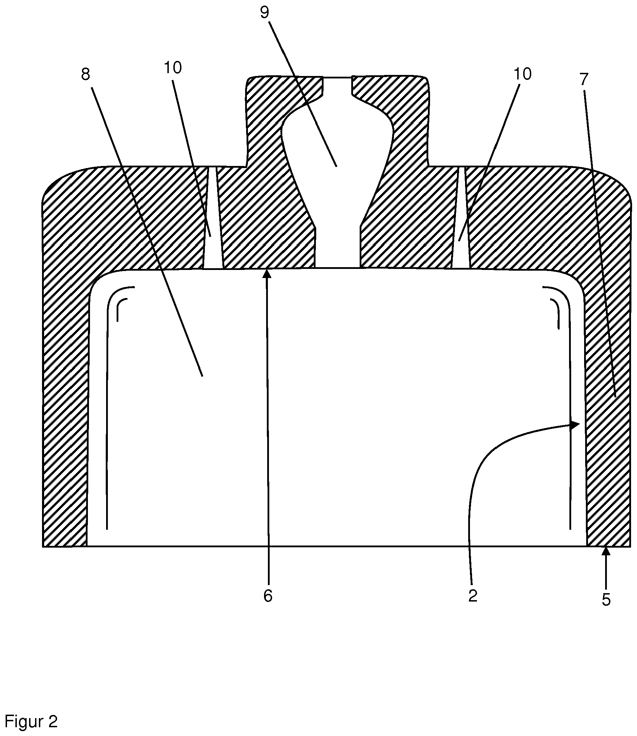

Figur 1 zeigt eine schematische perspektivische Ansicht eines unteren Teils einer erfindungsgemäßen Gießform zur Herstellung einer Tibia-Komponente eines Kniespacers und Figur 2 eine schematische Querschnittansicht eines oberen Teils einer erfindungsgemäßen Gießform zur Herstellung einer Tibia-Komponente eines Kniespacers. Wenn die beiden Teile zusammengesetzt werden, bilden Sie gemeinsam eine zweiteilige Gießform für eine Tibia-Komponente eines Kniespacers.

Die in den Figuren 1 und 2 gezeigten Gießform-Teile sind Hohlformen aus Edelstahl. Das untere Gießformteil (Figur 1) ist nach Art eines unten geschlossenen und oben offenen Behälters aufgebaut. Das untere Gießform-Teil weist eine Bodenfläche 1 auf, die ein Negativ der zu erzeugenden Gleitfläche der Tibiakomponente des beweglichen artikulierenden Kniespacers ist. Seitlich ist das untere Gießform-Teil durch Innenwände 2 begrenzt, die ebenfalls ein Negativ der zu erzeugenden Spacerform sind.

Aus einer Außenwand 3 des unteren Gießform-Teils ragt ein Kabel 4. Das Kabel 4 enthält elektrische Leitungen, die mit einem Heizdraht (nicht gezeigt) im Inneren des Gießformteils verbunden sind. Der Heizdraht und die elektrischen Leitungen sind selbstverständlich gegen die Gießform elektrisch isoliert. Alternativ zu einer elektrischen Beheizung des Gießform-Teils kann statt eines Kabels 4 auch ein Schlauch 4 vorgesehen sein, durch den eine beheizte Flüssigkeit zum Temperieren der Gießform fließen kann. Dazu sind innerhalb der Wandungen oder auf der Außenwand 3 der Gießform Rohre (nicht gezeigt) vorgesehen, durch die die beheizte Flüssigkeit (zum Beispiel Wasser) strömen kann. An einer anderen Stelle kann das Heizfluid durch einen weiteren Schlauch (nicht gezeigt) abgeführt werden.

Mit Hilfe der Fluidheizung oder der elektrischen Heizung kann die Gießform temperiert werden. Zum Einstellen einer konkreten Temperatur kann an wenigstens einer Stelle der Gießform ein Temperatursensor (nicht gezeigt) vorgesehen sein, der an eine Steuerung oder einen Regelkreis (nicht gezeigt) angeschlossen ist, mit dem die Heizleistung beziehungsweise die Temperatur und/oder die Durchflussmenge des Heizfluids (beispielsweise Wasser) gesteuert werden kann und damit die Temperatur der Gießform einstellbar ist.

Die obere Kante des unteren Gießform-Teils ist durch eine ebene Verbindungskante 5 gebildet. Die Verbindungskante 5 dient der Auflage auf ein oberes Gießform-Teil, wie es beispielhaft in Figur 2 dargestellt ist. Dort ist die Verbindungskante 5 als Unterseite einer Wandung 7 des oberen Gießform-Teils angeordnet. Die Gießform-Teile sind über die Verbindungskanten 5 dicht und zumindest Innen bündig miteinander verbindbar. Bevorzugt können die Gießform-Teile fest miteinander verbunden werden, beispielsweise durch eine lösbare Arretierung oder einen Verschluss (nicht gezeigt).

Das obere Gießform-Teil ist Innen auf der Oberseite durch eine Deckenfläche 6 begrenzt, die ein Negativ der der Tibia zugewandten Seite des Spacerteils darstellt. Die Innenwände 2, die Bodenfläche 1 und die Deckenfläche 6 bilden so ein Formnest 8 für das zu erzeugenden Spacerteil, das heißt das Tibiateil des zweiteiligen Kniespacers.

Ebenso wie das untere Gießform-Teil kann auch das obere Gießform-Teil geheizt, beziehungsweise temperiert werden.

Die Deckenfläche 6 ist durch einen Anguss 9 und mehrere Steiger 10 durchbrochen und nach Außen geöffnet. Die Steiger 10 und der Anguss 9 sind an den höchsten Punkten innerhalb der zusammengesetzten Gießform angeordnet. Durch den Anguss 9 wird die zusammengesetzte und auf 58 °C temperierte Gießform mit einem -18 °C kalten, unter Vakuum gemischten PMMA-Knochenzement (nicht gezeigt) gefüllt. Der Knochenzement kann dazu aus einem gewöhnlichen Tiefkühler oder einer Kühleinheit entnommen werden. Mit Hilfe der Steiger 10 wird sichergestellt, dass keine Lufteinschlüsse im Innenraum 8 der Gießform verbleiben.

Durch die höhere Temperatur der Innenwände 2, der Bodenfläche 1 und der Deckenfläche 6 beginnt die Polymerisation des PMMA-Knochenzements an diesen Oberflächen. Bei der Polymerisation wird Wärme freigesetzt, die zu einem fortschreiten der Polymerisationsfront ins Innere des aushärtenden Knochenzements im Inneren der Gießform führt. Dabei kommt es zu einer Schrumpfung des aushärtenden Knochenzements. Durch den Anguss 9 kann Knochenzement zum Ausgleich des Schrumpfungsverlusts nachgezogen werden, so dass sich in dem so gebildeten Spacer beziehungsweise Spacerteil keine Hohlräume bilden, beziehungsweise sich die Oberfläche des aushärtenden Knochenzements sich beim Aushärten nicht von den Innenflächen 1, 2, 6 der Gießform lösen.

Sobald der Knochenzement vollständig ausgehärtet ist, werden die beiden Gießform-Teile wieder voneinander gelöst und der fertige Spacer beziehungsweise das fertige Spacerteil kann entnommen werden. Überstände durch die Steiger 10 oder den Anguss 9 können mechanisch entfernt werden.

Zu den verwendbaren Knochenzementen und Kartuschen zum Mischen und Austragen der Knochenzemente sei auf die, aus dem Stand der Technik bekannten Ausführungsformen verwiesen.

Das mit den Figuren 1 und 2 gezeigte Ausführungsbeispiel lässt sich vom Fachmann ohne weiteres auf die zur Tibia-Komponente passende Femur-Komponente zur Bildung eines zweiteiligen artikulierenden Kniespacers übertragen. Die Erfindung soll aber nicht auf Kniespacer beschränkt verstanden werden. Mit dem gleichen Prinzip lasen sich auch andere zweiteilige aber auch einteilige Spacer herstellen. Dazu muss lediglich die Innere Form der Gießformteile (oder auch einer einteiligen Gießform) an den jeweils zu ersetzenden Knochen angepasst werden. Das Ausführungsbeispiel nach den Figuren 1 und 2 lässt sich also ohne weiteres für den Fachmann auf andere Spacer übertragen.

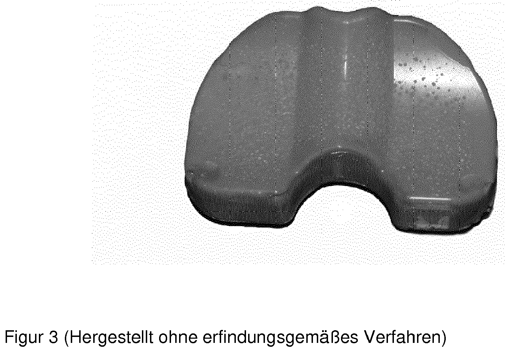

Figur 3 zeigt eine Abbildung einer Tibia-Komponente eines Kniespacers, der mit einem Verfahren ohne temperierte Gießform mit nicht elastischen Wandungen hergestellt wurde. Die Tibia-Komponente des Kniespacers wurde mit einer zweiteiligen Polyethylenform unter Verwendung des Knochenzements Palacos^{®} LV+G der Firma Heraeus Medical GmbH gegossen. Dabei hatte die Polyethylengießform eine Temperatur von 23 °C. Die Monomerflüssigkeit und das Zementpulver des Polymethylmethacrylatknochenzements hatten ebenfalls eine Temperatur von 23 °C. Nach der Aushärtung des Knochenzements wurde der Tibia-Spacer entformt. Figur 3 zeigt die Oberfläche des Spacerteils. Es sind deutlich Poren und andere Störungen der Oberfläche zu erkennen.

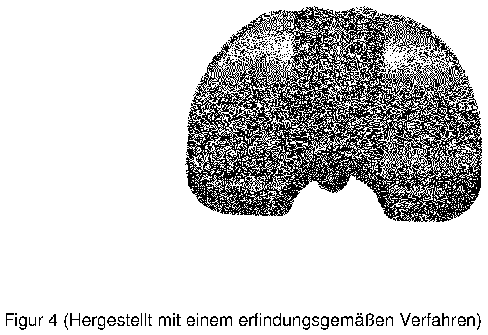

Figur 4 zeigt eine Abbildung einer Tibia-Komponente eines Kniespacers hergestellt mit einem erfindungsgemäßen Verfahren. Die Tibia-Komponente des Kniespacers wurde mit einer zweiteiligen Polyethylenform unter Verwendung des Knochenzementes Palacos^{®} LV+G der Firma Heraeus Medical GmbH gegossen. Dabei hatte die Polyethylengießform eine Temperatur von 55 °C. Die Monomerflüssigkeit und das Zementpulver des PolymethylmethacrylatKnochenzements hatten eine Temperatur von 23 °C. Nach der Aushärtung des Knochenzements wurde der Tibia-Spacer entformt. Figur 4 zeigt die Oberfläche des Spacerteils. Es ist deutlich erkennbar, dass der derart erzeugte Spacer keine Poren oder andere Oberflächenstörungen mehr aufweist.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Bodenfläche
- 2: Innenwand
- 3: Außenwand
- 4: Kabel / Schlauch
- 5: Verbindungskante
- 6: Deckenfläche
- 7: Wandung
- 8: Formnest / Innenraum der Gießform
- 9: Anguss
- 10: Steiger

## Patentansprüche

1. Verfahren zur Herstellung von Spacern aus einem Knochenzement aufweisend die folgenden chronologischen Schritte:
A) eine Gießform wird mittels einer Temperiereinrichtung auf eine erste Temperatur temperiert,
B) ein Zementteig mit einer Temperatur kleiner als die erste Temperatur der temperierten Gießform wird in die temperierte Gießform eingefüllt,
C) der Zementteig härtet innerhalb der Gießform zu einem Spacer aus und
D) nach der Aushärtung des Spacers wird die Gießform vom Spacer getrennt.

2. Verfahren nach Anspruch 1, **dadurch gekennzeichnet, dass**
als Zementteig ein Zementteig aus Polymethylmethacrylat verwendet wird, bevorzugt ein durch Vakuumeinwirkung entgaster Zementteig enthaltend Polymethylmethacrylat verwendet wird, und der erzeugte Spacer aus Polymethylmethacrylat besteht.

3. Verfahren nach Anspruch 1 oder 2, **dadurch gekennzeichnet, dass**
zumindest die den Spacer formende Oberfläche (1, 2, 6) der Gießform zu wenigstens 90%, bevorzugt zu wenigstens 99% aus einem nicht elastischen Material besteht, bevorzugt aus Polyethylen, Polypropylen, Polyamid und/oder Edelstahl, besonders bevorzugt aus dem Edelstahl 1.4401 und/oder dem Edelstahl 1.4404.

4. Verfahren nach einem der vorangehenden Ansprüche, **dadurch gekennzeichnet, dass** der Zementteig mit einer Temperatur, die zumindest 10 °C kleiner als die erste Temperatur des temperierten Gießform ist, in die temperierte Gießform eingefüllt wird, bevorzugt mit einer Temperatur, die zumindest 20 °C kleiner, besonders bevorzugt 20 °C bis 50 °C kleiner ist als die erste Temperatur des temperierten Gießform.

5. Verfahren nach einem der vorangehenden Ansprüche, **dadurch gekennzeichnet, dass** die Gießform auf eine erste Temperatur zwischen 40 °C und 65 °C temperiert wird, bevorzugt zwischen 45 °C und 60 °C, besonders bevorzugt zwischen 50 °C und 55 °C.

6. Verfahren nach einem der vorangehenden Ansprüche, **dadurch gekennzeichnet, dass** der Zementteig mit einer Temperatur zwischen -30 °C und 30 °C in die Gießform eingefüllt wird, bevorzugt zwischen -20 °C und 10 °C, besonders bevorzugt zwischen - 20 °C und 0 °C.

7. Verfahren nach einem der vorangehenden Ansprüche, **dadurch gekennzeichnet, dass** der Zementteig auf eine Temperatur kleiner als die erste Temperatur der temperierten Gießform gebracht wird, bevor er in die Gießform gefüllt wird.

8. Verfahren nach einem der vorangehenden Ansprüche, **dadurch gekennzeichnet, dass** der Zementteig mit einem Volumen, das zumindest 2 Vol% größer als das Formnest der Gießform ist, bevorzugt zumindest 5 Vol% größer ist, in die Gießform eingefüllt wird, wobei das zusätzliche Volumen zumindest vor dem Aushärten des Knochenzements in einem Anguss (9) der Gießform enthalten ist.

9. Verfahren nach einem der vorangehenden Ansprüche, **dadurch gekennzeichnet, dass** in die Gießform zur Armierung des Spacers mindestens ein Stahlkern eingelegt wird, dessen Temperatur mindestens 10 °C niedriger ist als die erste Temperatur der temperierten Gießform, bevorzugt mindestens 30 °C niedriger als die erste Temperatur der temperierten Gießform ist.

10. Verfahren nach Anspruch 9, **dadurch gekennzeichnet, dass**
der mindestens eine Stahlkern durch Abstandhalter in der Gießform derart gelagert wird, dass der Abstand zwischen der Innenseite der Gießform (1, 2, 6) und der Oberfläche des Stahlkern festgelegt ist, wobei die Abstandhalter eine Temperatur haben, die mindestens 10 °C niedriger ist als die erste Temperatur der temperierten Gießform, bevorzugt mindestens 30 °C niedriger als die erste Temperatur der temperierten Gießform ist.

11. Verfahren nach einem der vorangehenden Ansprüche, **dadurch gekennzeichnet, dass** der Knochenzement mindestens ein Antibiotikum und/oder ein Antiseptikum enthält, wobei bevorzugt zwei Antibiotika und/oder Antiseptika enthalten sind und ganz besonders bevorzugt drei Antibiotika und/oder Antiseptika enthalten sind.

## Claims

1. Method for producing spacers from a bone cement, comprising the following chronological steps:
A) controlling the temperature of a casting mould by means of a tempering device to a first temperature;
B) filling a cement dough, which has a temperature that is lower than the temperature of the temperature-controlled casting mould, into the temperature-controlled casting mould;
C) allowing the cement dough to cure in the casting mould to form a spacer; and
D) separating the casting mould from the spacer after the spacer is cured.

2. Method according to claim 1, **characterised in that**
a cement dough made of polymethylmethacrylate is used as cement dough, preferably a polymethylmethacrylate-containing cement dough that has been degassed by vacuum action, and the spacer thus produced consists of polymethylmethacrylate.

3. Method according to claim 1 or 2, **characterised in that**
at least the casting mould surface (1, 2, 6) forming the spacer consists of at least 90%, preferably at least 99% of a non-elastic material, preferably of polyethylene, polypropylene, polyamide and/or stainless steel, with stainless steel 1.4401 and/or stainless steel 1.4404 being particularly preferred.

4. Method according to any one of the preceding claims, **characterised in that**
the cement dough is filled into the temperature-controlled casting mould while it has a temperature at least 10 °C lower than the first temperature of the temperature-controlled casting mould, preferably a temperature at least 20 °C lower, particularly preferably 20 °C to 50 °C lower than the first temperature of the temperature-controlled casting mould.

5. Method according to any one of the preceding claims, **characterised in that**
the casting mould is temperature-controlled to a first temperature between 40 °C and 65 °C, preferably between 45 °C and 60 °C, particularly preferably between 50 °C and 55 °C.

6. Method according to any one of the preceding claims, **characterised in that**
the cement dough being filled into the casting mould has a temperature between -30 °C and 30 °C, preferably between -20 °C and 10 °C, particularly preferably between -20 °C and 0 °C.

7. Method according to any one of the preceding claims, **characterised in that**
the cement dough is brought to a temperature lower than the first temperature of the temperature-controlled casting mould before filling it into the casting mould.

8. Method according to any one of the preceding claims, **characterised in that**
the cement dough being filled into the casting mould has a volume that is at least 2 % by volume larger, preferably at least 5 % by volume larger, than the mould cavity, whereby the additional volume is present in a sprue (9) of the casting mould at least before the bone cement is cured.

9. Method according to any one of the preceding claims, **characterised in that**
at least one steel core is placed into the casting mould for reinforcement of the spacer, whereby the temperature of the steel core is at least 10 °C lower than the first temperature of the temperature-controlled casting mould, preferably at least 30 °C lower than the first temperature of the casting mould.

10. Method according to claim 9, **characterised in that**
the at least one steel core is suspended appropriately in the casting mould by means of separators such that the distance between the internal surface (1, 2. 6) of the casting mould and the surface of the steel core is defined, whereby the separators have a temperature that is at least 10 °C lower than the first temperature of the temperature-controlled casting mould, preferably at least 30 °C lower than the first temperature of the temperature-controlled casting mould.

11. Method according to any one of the preceding claims, **characterised in that**
the bone cement contains at least one antibiotic and/or one antiseptic, whereby it is preferred for two antibiotics and/or antiseptics to be present and particularly preferred for three antibiotics and/or antiseptics to be present.

## Revendications

1. Procédé de fabrication d'espaceurs à base d'un ciment osseux présentant les étapes chronologiques suivantes :
A) un moule est thermostaté à une première température au moyen d'un dispositif de thermostatation,
B) une pâte de ciment à une température inférieure à la première température du moule thermostaté est mise dans le moule thermostaté,
C) la pâte de ciment durcit à l'intérieur du moule pour donner un espaceur et
D) après le durcissement de l'espaceur, le moule est séparé de l'espaceur.

2. Procédé selon la revendication 1, **caractérisé en ce**
**qu'**en tant que pâte de ciment, on emploie une pâte de ciment à base de polyméthyl méthacrylate, de préférence, on emploie une pâte de ciment dégazée sous l'effet du vide contenant du polyméthyl méthacrylate, et l'espaceur créé est constitué de polyméthyl méthacrylate.

3. Procédé selon la revendication 1 ou 2, **caractérisé en ce**
**qu'**au moins la surface (1, 2, 6) formant l'espaceur du moule est constituée à au moins 90 %, de préférence à au moins 99 % d'un matériau non élastique, de préférence à base de polyéthylène, de polypropylène, de polyamide et/ou d'acier inoxydable, de manière particulièrement préférée à base de l'acier inoxydable 1.4401 et/ou de l'acier inoxydable 1.4404.

4. Procédé selon l'une des revendications précédentes, **caractérisé en ce que** la pâte de ciment est mise dans le moule thermostaté à une température qui est inférieure d'au moins 10 °C à la première température du moule thermostaté, de préférence avec une température qui est inférieure d'au moins 20 °C, de manière particulièrement préférée, inférieure de 20 °C à 50 °C à la première température du moule thermostaté.

5. Procédé selon l'une des revendications précédentes, **caractérisé en ce que** le moule est thermostaté à une première température entre 40 °C et 65 °C, de préférence entre 45 °C et 60 °C, de manière particulièrement préférée entre 50 °C et 55 °C.

6. Procédé selon l'une des revendications précédentes, **caractérisé en ce que** la pâte de ciment est mise dans le moule à une température entre -30 °C et 30 °C, de préférence entre -20 °C et 10 °C, de manière particulièrement préférée entre -20 °C et 0 °C.

7. Procédé selon l'une des revendications précédentes, **caractérisé en ce que** la pâte de ciment est apportée à une température inférieure à la première température du moule thermostaté avant qu'elle ne soit mise dans le moule.

8. Procédé selon l'une des revendications précédentes, **caractérisé en ce que** la pâte de ciment est mise dans le moule avec un volume qui est au moins supérieur de 2 % en volume à l'intérieur du moule, de préférence supérieur d'au moins 5 % en volume, où le volume supplémentaire est inclus au moins avant le durcissement du ciment osseux dans un culot (9) du moule.

9. Procédé selon l'une des revendications précédentes, **caractérisé en ce qu'**au moins un noyau en acier est déposé dans le moule pour le renforcement de l'espaceur, dont la température est au moins de 10 °C inférieure à la première température du moule thermostaté, de préférence au moins de 30 °C inférieure à la première température du moule thermostaté.

10. Procédé selon la revendication 9, **caractérisé en ce que**
l'au moins un noyau en acier est bloqué dans le moule par des cales d'espacement de telle manière que la distance entre la face interne du moule (1, 2, 6) et la surface du noyau en acier est définie, où les cales d'espacement ont une température qui est inférieure d'au moins 10 °C à la première température du moule thermostaté, de préférence inférieure d'au moins 30 °C à la première température du moule thermostaté.

11. Procédé selon l'une des revendications précédentes, **caractérisé en ce que** le ciment osseux contient au moins un antibiotique et/ou un antiseptique, où, de préférence, deux antibiotiques et/ou antiseptiques sont présents, et de manière particulièrement préférée, trois antibiotiques et/ou antiseptiques sont présents.
